(19) Europäisches Patentamt

European Patent Office

Office européen des brevets

(11) **EP 1 536 800 B1**

(12) **EUROPEAN PATENT SPECIFICATION**

(45) Date of publication and mention
of the grant of the patent:
**04.01.2006 Bulletin 2006/01**

(51) Int Cl.:
*A61K 31/52* (2006.01)    *A61K 31/711* (2006.01)
*A61P 31/18* (2006.01)

(21) Application number: **03733801.9**

(22) Date of filing: **24.06.2003**

(86) International application number:
**PCT/SE2003/001100**

(87) International publication number:
**WO 2004/002433 (08.01.2004 Gazette 2004/02)**

(54) **SYNERGISTIC INTERACTION OF ABACAVIR AND ALOVUDINE**

SYNERGISTISCHE WECHSELWIRKUNG VON ABACAVIR UND ALOVUDIN

INTERACTION SYNERGIQUE D'ABACAVIR ET D'ALOVUDINE

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **27.06.2002 SE 0202022
06.11.2002 EP 02024744**

(43) Date of publication of application:
**08.06.2005 Bulletin 2005/23**

(73) Proprietor: **MEDIVIR AB
141 44 Huddinge (SE)**

(72) Inventor: **MARDH, Göran
S-114 59 Stockholm (SE)**

(74) Representative: **Dehmel, Albrecht
Dehmel & Bettenhausen
Patentanwälte,
Herzogspitalstrasse 11
80331 München (DE)**

(56) References cited:
**EP-A1- 0 346 132        EP-A2- 0 434 450
WO-A1-00/16779        WO-A1-88/00050
WO-A2-00/43014        US-A- 5 571 798**

## Description

Technical Field

This invention relates to the unexpected level of synergy exhibited between the HIV and HBV antivirals alovudine and abacavir against multiresistant HIV. The invention provides novel pharmaceutical preparations comprising the two agents in admixture or separately for concomitant or sequential administration and methods of treatment involving them.

Background to the Invention

Alovudine (3'-deoxy-3'-fluorothymidine, FLT) is described in WO88/00088 as an antiviral active against HIV and HBV. Alovudine is a prodrug which is converted in vivo to the active triphosphate.

Abacavir ((1R,4S)-9-[4-(hydroxymethyl)-2-cyclopenten-1-yl]guanine, carbovir) is described in EP 0434450 as having potent activity against HIV and HBV. Abacavir is also a prodrug which is converted in vivo to the active triphosphate.

Alovudine and abacavir have each exhibited modest synergy with certain selected nucleosides, especially in in vitro tests (see for example US 5,571,798 and WO 00/16779). However, we have now discovered in the clinical context that the particular combination of alovudine and abacavir produces a degree of antiviral synergy which is significantly greater than the usual level of synergy shown by the respective active agent.

Brief description of the invention

A first aspect of the invention provides a pharmaceutical preparation comprising a synergistic combination of abacavir and alovudine and a pharmaceutical carrier therefor.

A further aspect of the invention provides the use of abacavir and alovudine together for the manufacture of a medicament for the treatment of HBV or especially multiresistant HIV, wherein the use comprises combined, concomitant or sequential administration of alovudine and abacavir.

The invention further provides a patient pack comprising alovudine and/or abacavir and an information insert containing directions on the use of both alovudine and abacavir together in combination.

The unexpectedly profound degree of antiviral synergy of the invention provides such benefits as more complete viral suppression, viral suppression over longer periods, limits the emergence of drug-escape mutations and thus the development of multiresistant HIV and HBV and allows better management of drug related toxicities. The use of this drug combination may, in some circumstances decrease the number of pills taken by the patient and therefore increase patient compliance.

It will be appreciated that the alovudine and abacavir combination of the invention may be administered simultaneously, either in the same or different pharmaceutical composition, or sequentially. In the case of sequential administration, the delay in administering the second active ingredient should not be such as to lose the synergistic benefit of the invention. Typically sequential administration will not involve delays of greater than 12 hours, preferably less than 1 hour, such as before and after a meal.

Due its good tolerability, once day dosing and very small dosage/day, alovudine can advantageously be administered as an add-on to existing HAART regimes, such as therapies comprising one or two nucleoside analogues and a protease inhibitor and/or one or more NNRTIs. Such permutations can be chosen from conventional HIV antivirals such as 3TC, ddI (2',3'-dideoxyinosine), nevirapine, delavirdine, efavirenz, ritonavir, kaletra, saquinavir, amprenavir, amprenavir phosphate, indinavir etc. Preferably the preexisting regime or concomitant antiviral does not include d4T.

For ease of administration the alovudine and abacavir are conveniently presented in the same unit dosage form, such as a capsule or tablet or in a fluid containing appropriate concentrations of the two active agents.

The amount of alovudine and abacavir necessary for suppression of HIV or HBV will, of course vary from patient to patient and is ultimately at the discretion of the medical practitioner taking account of such well known factors as body weight, route of administration, concomitant medication, ag, gender and general condition and the nature and severity of the disease.

In general abacavir is dosed in the range of about 3 to about 120 mg/kg/day such as 1-90 mg/kg/day, preferably 5-60 mg/kg/day. Preferably the abacavir is present in an amount of 200-800 mg per unit dose, more preferably 300-500 mg per unit dose.

In general alovudine is dosed in the range of about 0.001-0.5 mg/kg/day, preferably 0.005-0.15 mg/kg/day. Favoured regimes thus include 0.01-0.5 mg/kg/day, such as 0.05-0.12 mg/kg/day. Preferably the alovudine is present in an amount of 0.1-20 mg per unit dose, such as 0.5-10 mg per unit dose, especially 0.5-5 mg/unit dose, such as 2-5 mg per unit dose.

Alovudine and abacavir are conveniently administered once or twice a day, especially once per day.

The alovudine and abacavir are conveniently administered and/or presented in a weight ratio corresponding

to their respective ED$_{50}$, for example in the ratio 1-10:200-800

Abacavir is commercially available and its production is extensively described in the patent and academic literature. Alovudine is conveniently prepared as described in EP 495 225 and EP 470 355.

Abacavir and alovudine, particularly at the dosage rates herein described, are readily formulated in conventional pharmaceutical carriers and with conventional excipients. The compounds of the invention are particularly suited to oral administration, but may also be administered rectally, vaginally, nasally, topically, transdermally or parenterally, for instance intramuscularly, intravenously or epidurally. The compounds may be administered alone, for instance in a capsule, but will generally be administered in conjunction with a pharmaceutically acceptable carrier or diluent. The invention extends to methods for preparing a pharmaceutical composition comprising bringing alovudine and abacavir in conjunction or association with a pharmaceutically acceptable carrier or vehicle.

Oral formulations are conveniently prepared in unit dosage form, such as capsules or tablets, employing conventional carriers or binders such as magnesium stearate, chalk, starch, lactose, wax, gum or gelatin. Liposomes or synthetic or natural polymers such as HPMC or PVP may be used to afford a sustained release formulation. Alternatively the formulation may be presented as a nasal or eye drop, syrup, gel or cream comprising a solution, suspension, emulsion, oil-in-water or water-in-oil preparation in conventional vehicles such as water, saline, ethanol, vegetable oil or glycerine, optionally with flavourant and/or preservative and/or emulsifier.

Brief description of the drawings

An embodiment of the invention will be illustrated by way of example only with reference to the drawings in which:

Figure 1 depicts median reduction in viral load in patients treated with the synergistic combination of the invention comprising alovudine added to an abacavir-containing regimen; in contrast to patients treated with alovudine and a non-abacavir-containing regimen.

Figure 2 depicts reduction in viral load in a comparative experiment in which an alovudine /ddl-containing regimen is plotted beside an alovudine/non-ddl regimen.

Figure 3 depicts HIV suppression as a function of antiviral concentration for alovudine, abacavir or the 1:200 combination.

Detailed description of the invention

Example 1 Clinical Trial

A phase IIa trial was performed with 15 patients failing their current NRTI-containing regimens. Each patient had HIV RNA > 1000 cp/ml, with at least 2 mutations in the viral RT induced by previous viral therapy, as established by genotypic assay. Patients had a baseline viral load of 3.93 log$_{10}$ cp/ml.

The patients were administered qd 7.5 mg alovudine in a conventional carrier in addition to their current regimen for four weeks. The current regimes included various permutations of 3-5 HIV antivirals selected from 3TC, ddl, D4T, nevirapine, DMP, ritonavir, kaletra, saquinavir, amprenavir and indinavir, administered in their conventional dosage forms and regimens. Antiviral load evaluation was performed weekly and then four weeks after discontinuation of alovudine. The alovudine addition was generally well tolerated and there was no withdrawal from therapy and no serious adverse events. A transient mean increase in CD4 counts of +52 counts/mm$^3$ was detected.

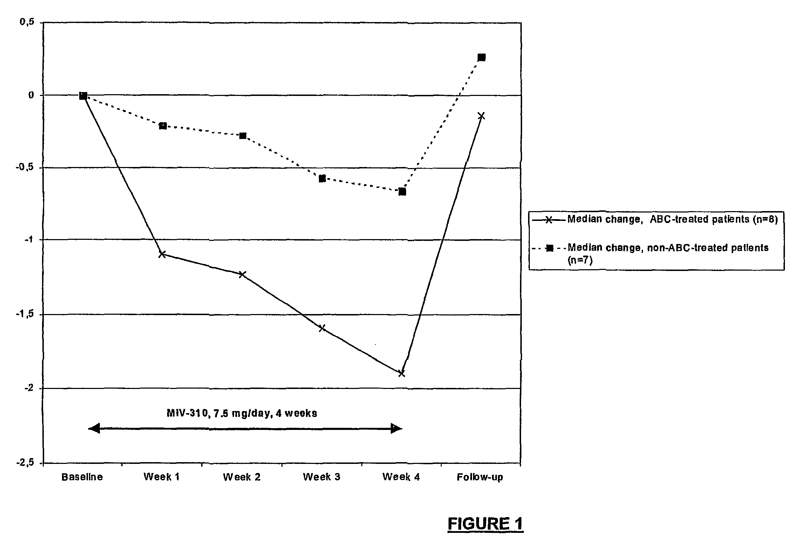

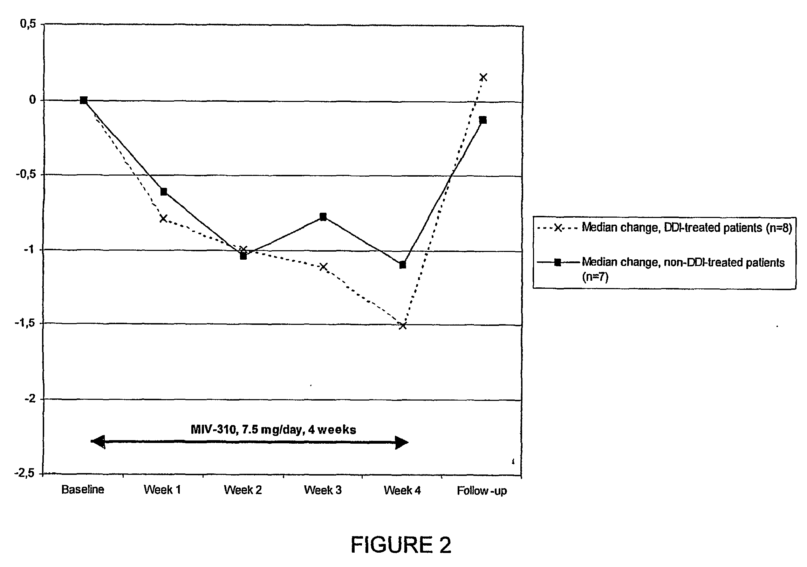

The results are plotted in Figures 1 and 2. Referring initially to Figure 1, it will be apparent that the alovudine/abacavir-containing regimen results in significantly lower median viral loads compared to patients administered with alovudine but whose concomitant regimen did not include abacavir. This profound reduction in viral load with alovudine/abacavir of the invention is surprising when contrasted with the performance of alovudine and ddl (didanosine) regimes in Figure 2, plotted against non-ddl containing regimens. Alovudine and ddl show clear synergy in *in vitro* tests (Cox et al AIDS Res Hum Retrovir. 1994 (12):1275-9). As is seen in Figure 2, this known synergy translates in the clinical setting to a 0.2-0.3 log reduction. In contrast the combination of the invention consistently resulted in 1-1.5 log reductions, which is a quantum jump in synergy bearing in mind the logarithmic scale used.

As this clinical trial was performed as an add-on therapy to the patients preexisting regime, it will be appreciated that the alovudine qd was administered in a separate dosage form to the abacavir (typically bd) and other antivirals (typically administered 2-4 times per day).

Example 2 Cell culture experiments

The antiviral effects of MIV-310 in combination with other NRTIs on HIV-1 replication in MT4 cells were examined by the median effect method (Chao and Talalay 1983). The assay was performed in microtiter plates with each well

containing $10^5$ cells and 20-50 tissue culture infective doses of HIV-1, IIIb. Each drug alone and a mixture of the drugs in a constant ratio were serially diluted in 2-fold steps and added to the wells in quadruplicates. The constant ratio is selected to reflect the relative potency of the drugs. For example the potency of abacavir in cell culture is 200 times less than the potency of alovudine. Accordingly, within a mixture, the relative contribution of abacavir would be drowned by the potency of alovudine if the mixture was equimolar. At a ratio of 1:200 abacavir:alovudine the respective contributions of the two drugs become apparent and the additional activity due to synergy is measurable. The anti-viral effect was measured day 5 using the XTT colorimetric method.

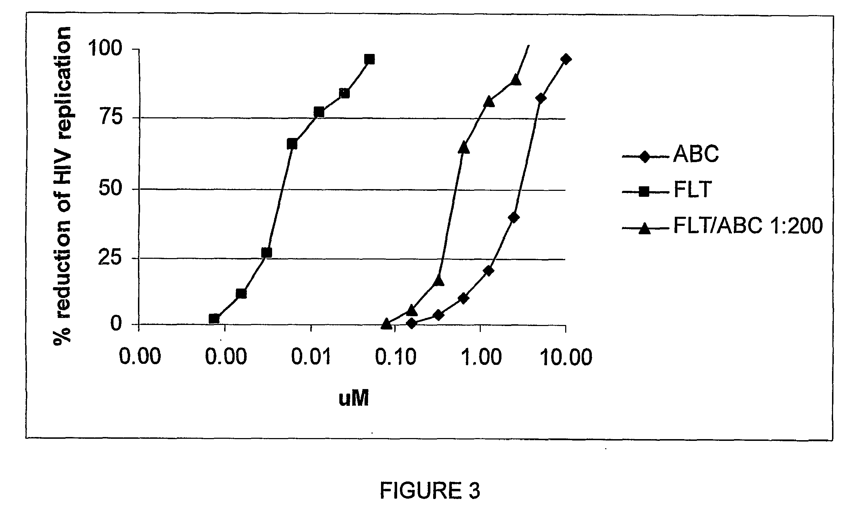

Figure 3 plots antiviral activity against concentration for alovudine (abbreviated to FLT on this graph) and abacavir (abbreviated to ABC on this graph) or and the mixture of the two at 1:200. Dissecting the points of the alovudine: abacavir 1:200 curve, especially those around the $ED_{50}$, it is readily apparent that the antiviral activity of the mixture is greater than the sum of the contributions of the respective parts. For example, the triangle at 65% inhibition at 0.63uM reflects a mixture where the abacavir component is at 0.63 micromolar and the alovudine component is 1/200 of that, ie 0.003 micromolar. However, abacavir alone (diamonds) at 0.63 uM produces an inhibition of just 10%, whereas alovudine alone (squares) at 0.003 uM produces an inhibition of 27%. The sum of the components is thus 37%. In comparison the mixture at these same concentrations produces 60% inhibition.

The effective dose giving 50% reduction of virus replication, $ED_{50}$ (or $ED_{75}$, $ED_{90}$ etc) was calculated for single drugs and for the combinations and the Combination Index calculated by median effect (ibid) to equal

$$D_A / Dx_A + D_B / Dx_B + \alpha(D_A D_B)/(Dx_A Dx_B)$$

where $Dx_A$ is the concentration which provides a given activity of the compound A alone, $D_A$ is the concentration of A in the mixture to provide the same inhibition, $Dx_B$ and $D_B$ apply similarly for compound B, and alpha is zero if the compounds are mutually exclusive or 1 if mutually exclusive.

A combination index (CI) less than 1 indicates synergism, CI equal to zero indicates addition and a CI greater than 1 indicates antagonism. The combination indices for the combination of the invention in comparison to prior art combinations is tabulated in Table 1 below. Once again, the combinations have been tested at molar ratios reflecting their relative $ED_{50}$s in cell culture.

Table 1

| Combination | Molar ratio | Combination Index | | |
| --- | --- | --- | --- | --- |
| | | 50% inhibition | 75% inhibition | 90% inhibition |
| FLT + stavudine (d4T) | 1:40 | 0.94 | 0.96 | 0.99 |
| FLT + zidovudine (AZT) | 1:1 | 0.70 | 0.71 | 0.72 |
| FLT + didanosine (ddl) | 1:250 | 0.95 | 0.76 | 0.61 |
| FLT + abacavir (ABC) | 1:200 | 0.74 | 0.57 | 0.47 |

The preferred alovudine combination in the prior art (US 5,571,798) is AZT/alovudine which in this experiment produces an average CI of 0.71 across the three levels tested. In contrast the combination of the invention produces an average CI of 0.59, ie a significantly more profound degree of synergy than is demonstrated in the prior art combination. Similarly, another favoured combination in US 5,571,798, ddl in conjunction with FLT produces an average CI of 0.77, once again showing that the combination of invention produces synergies significantly more intense than the levels of synergy previously seen.

As the goal of most antiviral therapies is to maintain serum trough levels corresponding at least to an $ED_{90}$ to prevent the development of resistant mutants, synergistic performance at the higher end of the spectrum has the greatest advantage. It will be apparent from Table 1 that the relative CI between the combination of the invention and prior art combinations is even better at $ED_{90}$ compared to the average figures cited in the immediately preceding paragraph.

Consistent with the clinical trial of Example 1, Table 1 indicates that stavudine (d4T) is a less favoured component to combine with alovudine and did not exhibit any significant degree of synergy as measured by CI.

## Claims

1. A pharmaceutical preparation comprising a synergistic combination of abacavir and alovudine and a pharmaceutical carrier therefor.

2. A preparation according to claim 1 wherein the alovudine is present in an amount of 1-10 mg per unit dose.

3. A preparation according to claim 2 wherein the alovudine is present in an amount of 0.5-7.5 mg per unit dose.

4. A preparation according to claim 3 wherein the alovudine is present in an amount of 0.5-5 mg per unit dose.

5. A preparation according to claim 1, wherein the abacavir is present in an amount of 200-800 mg per unit dose.

6. A preparation according to claim 5, wherein the abacavir is present in an amount of 300-500 mg per unit dose.

7. A preparation according to claim 1, wherein the alovudine and abacavir are present in a weight ratio corresponding to their respective $ED_{50}$.

8. A preparation according to claim 1, wherein the alovudine and abacavir are present in the ratio 1-10:200-800.

9. A patient pack comprising alovudine and/or abacavir and an information insert containing directions on the use of both alovudine and abacavir together in combination.

10. Use of abacavir and alovudine together in the manufacture of a pharmaceutical for the treatment of multiresistant HIV, wherein the pharmaceutical is formulated to allow simultaneous, combined or sequential administration of alovudine and abacavir.

11. Use according to claim 10, wherein abacavir and alovudine are in the form of the preparation of any of claims 1-8.

## Patentansprüche

1. Eine pharmazeutische Zubereitung umfassend eine synergistische Kombination von Abacavir und Alovudine und einem pharmazeutischen Träger dafür.

2. Die Zubereitung nach Anspruch 1, wobei das Alovudine in einer Menge von 1-10 mg pro Dosiseinheit vorliegt.

3. Die Zubereitung nach Anspruch 2, wobei das Alovudine in einer Menge von 0,5-7,5 mg pro Dosiseinheit vorliegt.

4. Die Zubereitung nach Anspruch 3, wobei das Alovudine in einer Menge von 0,5-5 mg pro Dosiseinheit vorliegt.

5. Die Zubereitung nach Anspruch 1, wobei das Abacavir in einer Menge von 200-800 mg pro Dosiseinheit vorliegt.

6. Die Zubereitung nach Anspruch 5, wobei das Abacavir in einer Menge von 300-500 mg pro Dosiseinheit vorliegt.

7. Die Zubereitung nach Anspruch 1, wobei das Alovudine und das Abacavir in einem Gewichtsverhältnis korrespondierend zu ihren respektiven $ED_{50}$ vorliegen.

8. Die Zubereitung nach Anspruch 1, wobei das Alovudine und das Abacavir in einem Verhältnis 1-10:200-800 vorliegen.

9. Eine Arzneimittelpackung für Patienten umfassend Alovudine und/oder Abacavir und einen Beipackzettel beinhaltend Anweisungen zur Verwendung von Alovudine und Abacavir in Kombination.

10. Gemeinsame Verwendung von Abacavir und Alovudine in der Herstellung eines Arzneimittels zur Behandlung von multiresistentem HIV, wobei das Arzneimittel so formuliert, dass es die simultane, kombinierte oder sequenzielle Verabreichung von Alovudine und Abacavir gestattet.

**EP 1 536 800 B1**

**11.** Verwendung nach Anspruch 10, wobei Abacavir und Alovudine in Form der Zubereitung nach einem der Ansprüche 1 bis 8 vorliegen.

**Revendications**

**1.** Préparation pharmaceutique comprenant une combinaison synergique d'abacavir et d'alovudine, et un support pharmaceutique.

**2.** Préparation selon la revendication 1, dans laquelle l'alovudine est présente dans une quantité de 1 à 10 mg par dose unitaire.

**3.** Préparation selon la revendication 2, dans laquelle l'alovudine est présente dans une quantité de 0,5 à 7,5 mg par dose unitaire.

**4.** Préparation selon la revendication 3, dans laquelle l'alovudine est présente dans une quantité de 0,5 à 5 mg par dose unitaire.

**5.** Préparation selon la revendication 1, dans laquelle l'abacavir est présent dans une quantité de 200 à 800 mg par dose unitaire.

**6.** Préparation selon la revendication 5, dans laquelle l'abacavir est présent dans une quantité de 300 à 500 mg par dose unitaire.

**7.** Préparation selon la revendication 1, dans laquelle l'alovudine et l'abacavir sont présents dans un rapport pondéral correspondant à leur $ED_{50}$ respective.

**8.** Préparation selon la revendication 1, dans laquelle l'alovudine et l'abacavir sont présents dans un rapport 1-10 : 200-800.

**9.** Conditionnement pour patient comprenant de l'alovudine et/ou de l'abacavir et une notice d'information contenant des instructions sur l'utilisation de l'alovudine et de l'abacavir conjointement en combinaison.

**10.** Utilisation d'abacavir et d'alovudine conjointement dans la fabrication d'un médicament pour le traitement de VIH multirésistant, dans laquelle le médicament est formulé de façon à permettre une administration simultanée, combinée ou séquentielle d'alovudine et d'abacavir.

**11.** Utilisation selon la revendication 10, dans laquelle l'abacavir et l'alovudine sont sous la forme d'une préparation de l'une quelconque des revendications 1 à 8.

**FIGURE 1**

FIGURE 2

FIGURE 3

EP 1 536 800 B1